# EUROPEAN PATENT APPLICATION

(11) **EP 2 281 807 A1**
(43) Date of publication of application: **09.02.2011**
(21) Application number: 09178811.7
(22) Date of filing: 11.12.2009
(51) Int. Cl.: C07C 231/24, C07C 237/46

(54) **Decolorizing 5-amino-N,N'-bis(2,3-dihydroxypropyl)-2,4,6-triiodoisophthalamide, a contrast media intermediate**

(30) Priority: 21.07.2009 US 227083 P
(71) Applicant: GE Healthcare AS, 0401 Oslo (NO)
(72) Inventor: Ingvoldstad, Odd, Einar, 4521 Spangereid (NO)
(74) Representative: Wulff, Marianne Weiby

(57) **Abstract**

This invention relates to an improved method for the synthesis of 5-amino-N,N'-bis(2,3-dihydroxypropyl)-2,4,6-triiodoisophthalamide (Compound B), an intermediate in the industrial preparation of non-ionic X-ray contrast agents. In particular, it relates to a process for decolorizing said intermediate.

## Description

### TECHNICAL FIELD

This invention relates generally to synthesis of non-ionic X-ray contrast agents. It further relates to an improved method for the synthesis of 5-amino-N,N'-bis(2,3-dihydroxypropyl)-2,4,6-triiodoisophthalamide (compound B), an intermediate in the industrial preparation of non-ionic X-ray contrast agents. In particular, it relates to a process for decolorizing said intermediate.

### BACKGROUND OF THE INVENTION

Non-ionic X-ray contrast agents constitute a very important class of pharmaceutical compounds produced in large quantities. 5-[N-(2,3-dihydroxypropyl)-acetamido]-N,N'-bis(2,3-dihydroxypropyl)-2,4,6-triiodo-isophthalamide ("iohexol"), 5-[N-(2-hydroxy-3-methoxypropyl)acetamido]-N,N'-bis(2,3-dihydroxypropyl)-2,4,6-triiodo-isophthalamide ("iopentol") and 1,3-bis(acetamido)-N,N'-bis[3,5-bis(2,3-dihydroxypropyl-aminocarbonyl)-2,4,6-triiodophenyl]-2-hydroxypropane ("iodixanol") are important examples of such compounds. They generally contain one or two triiodinated benzene rings.

For example, iodixanol, marketed under the trade name Visipaque®, is one of the most used agents in diagnostic X-ray procedures. It is produced in large quantities by GE Healthcare in Lindesnes, Norway. The industrial production of iodixanol involves a multistep chemical synthesis as shown in Scheme 1 below. To reduce the cost of the final product, it is critical to optimize each synthetic step. Even a small improvement in reaction design can lead to significant savings in a large scale production.

In the acetylation step of the industrial scale synthesis 5-amino-N,N'-bis(2,3-dihydroxypropyl)-2,4,6-triiodoisophthalamide (Compound B), is acetylated to produce 5-acetylamino-N,N'-bis(2,3-dihydroxypropyl)-2,4,6-triiodoisophthalamide (Compound A). Iodixanol may further be prepared by dimerization of Compound A. Compound B may hence be a key intermediate in the synthesis of the X-ray contrast agent iodixanol, but also for iohexol and ioversol, marketed under the names Visipaque™, Omnipaque™, and Optiray™, respectively.

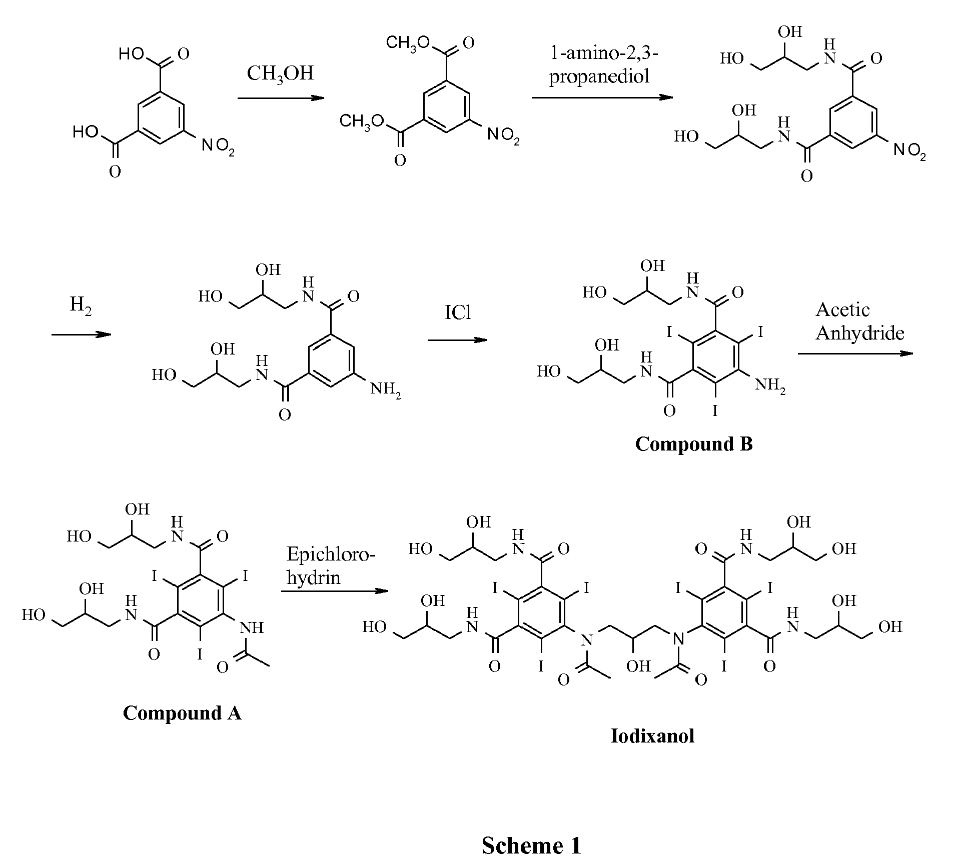

Scheme 1 shows the Compound B, how this is prepared and how this can react to form iodixanol.

### SUMMARY OF THE INVENTION

The present invention provides an industrial process for producing 5-amino-N,N'-bis(2,3-dihydroxypropyl)-2,4,6-triiodoisophthalamide ("Compound B") and decolorizing this. Compound B is an important intermediate in the process for preparing several non-ionic X-ray contrast agents. Specifically, the invention is directed to a process for preparing a colourless reaction mixture comprising Compound B by adding sodium dithionite to a solution comprising Compound B.

### DETAILED DESCRIPTION OF THE INVENTION

Compound B can be made by iodination of 5-amino-N,N'-bis(2,3-dihydroxypropyl)-isophthalamide or its hydrochlorid as shown in Scheme 2:

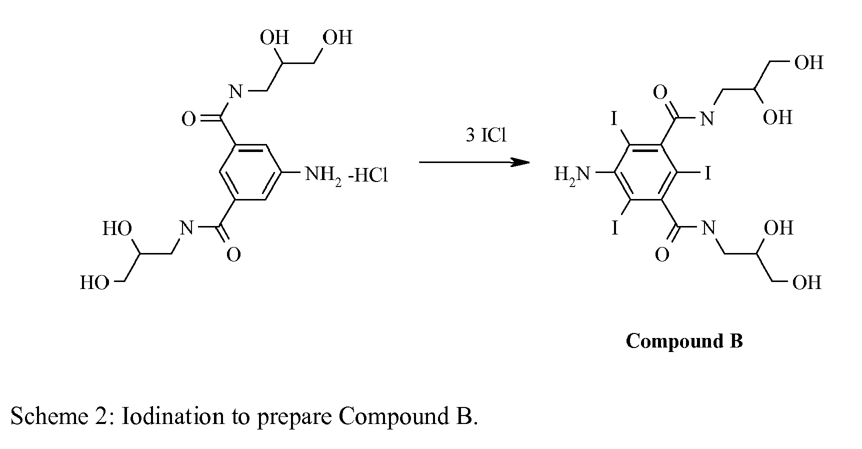

Iodine chloride (ICI) is used as the iodination reagent in the reaction. Excess ICl is reacted with sodium bisulphite (Na₂S₂O₅) after complete reaction. This is done to ensure that no iodination agent, iodine chloride, is present when further purification begins, since ICl is very reactive, especially in oxidation reactions, and as by products may be formed. The reaction with sodium bisulphite bleaks the reaction mixture from a dark red/brown/violet colour to a more pale appearance. However, some red/yellow colour is still observed. The intensity of the colour of the reaction product may vary from batch to batch. It has unfortunately been found that this colour follows through the subsequent reaction steps and may miscolour the final product, e.g. iohexol, iodixanol or ioversol. It is a by-product from the iodination reaction that is present in the reaction mixture in an amount of less than 1 %, as analysed by HPLC, that causes miscolouring of the reaction mixture. The by-product is a so-called azo dimer of Compound B.

In the present invention, we have however found that addition of a minor amount of sodium dithionite (Na₂S₂O₄) to Compound B solves the problem of miscolouring. Sodium dithionite is a stronger reduction agent than sodium bisulphite, and is able to reduce the azo dimer of Compound B to the corresponding hydrazo dimer. The azo dimer is a coloured by-product. Scheme 3 shows the reaction of the azo dimer of Compound B to the corresponding hydrazo dimer.

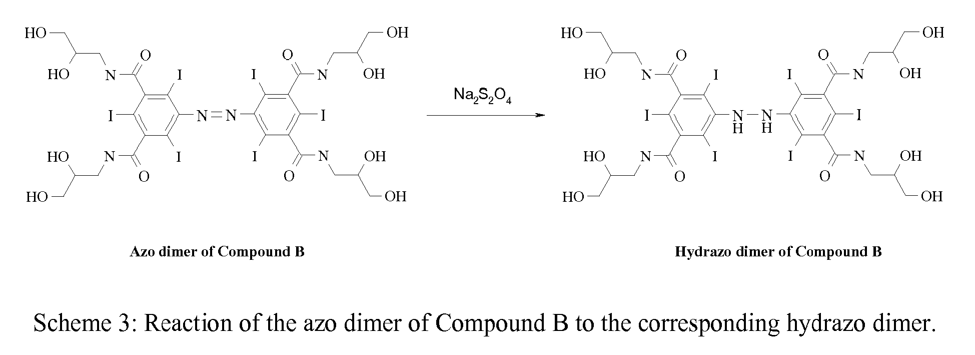

The azo dimer has a strongly yellow to red colour, while the hydrazo dimer is colourless due to the break in the double bond conjugation over the nitrogen bridge. The hydrazo dimer can be separated from Compound B by means of crystallization.

Thus, in a first aspect the invention provides a process for preparing a solution free of any red colour comprising 5-amino-N,N'-bis(2,3-dihydroxypropyl)-2,4,6-triiodoisophthalamide ("Compound B") by:
i) iodinating 5-amino-N,N'-bis(2,3-dihydroxypropyl)-isophthalamide or its hydrochloride to prepare a process solution comprising Compound B;
ii) adding sodium dithionite to the process solution of i).

In step i) 5-amino-N,N'-bis(2,3-dihydroxypropyl)-isophthalamide or its hydrochloride is typically dissolved in water or an alcoholic solvent, like methanol, or in a mixture of these. pH is preferably adjusted before iodinating, most preferably to about 3, by adding a base such as sodium hydroxide. The iodination is preferably done by iodine chloride (3 mole equivalents). The iodinating step may take place by adding the iodinating agent in several portions such as 1-5 portions, more preferably 3-4 portions, with pH adjustments to pH 2-3 before each addition.

The process optionally further includes the steps of adding a reactant to the solution i) to react with excess iodination agent, i.e. an agent such as sodium bisulphite may be added to the solution of i) after complete iodination, but before addition of sodium dithionite. Typically, 2-5 mole %, or more preferably 3-4 mole % sodium bisulphite is added, based on the amount of 5-amino-N,N'-bis(2,3-dihydroxypropyl)-isophthalamide or its hydrochloride, to react with excess iodination agent.

The pH should be kept above about 4 when sodium dithionite is added to the process solution, since solid sulphur may be formed at lower pH. Hence, the process may include the addition of a base, such as sodium hydroxide, to the solution of i) to adjust pH to above 4, such as to 4-6, before step ii).

In step ii) 1-10 mole %, more preferably 3-6 mole % sodium dithionite is added, based on the amount of 5-amino-N,N'-bis(2,3-dihydroxypropyl)-isophthalamide or its hydrochloride.

Further, the process optionally includes crystallizing Compound B directly from the process solution of ii) including filtering, washing and drying the crystallized product. A crystallized product of Compound B free of any red colour is obtained and this may be used in further reactions steps to produce a colourless non-ionic X-ray contrast agent.

In a second aspect the invention provides a solution of Compound B free of any red colour, and in a third aspect the invention provides a crystallized product of Compound B free of any red colour. The red colour free solution and crystallized product are prepared by the process as described in the first aspect.

The invention is illustrated further by the following examples that are not to be construed as limiting the invention in scope to the specific procedures described in them.

### EXAMPLES

### Example 1:

A solution of the azo dimer of Compound B in a 1:1 mixture of water and methanol (19 mg in 10 mL) was prepared. A small amount of sodium dithionite (about 50 mg) was added to 2 mL of the prepared solution at ambient temperature, resulting in immediate colour change from dark red to colourless.

### Example 2:

5-amino-N,N'-bis(2,3-dihydroxypropyl)-isophthalamide hydrochloride (754 kg, 2.07 kmole) is dissolved in water (2430 L) at ambient temperature. pH is adjusted to about 3 with aqueous sodium hydroxide (50 w/w %) and tri-iodinated with iodine chloride added in four portions (totally 6.37 kmole) at 65-80°C. pH is adjusted with aqueous sodium hydroxide to 2-3 before each addition of iodine chloride. Excess iodine chloride is reacted with sodium bisulphate (15-20 kg, 0.08-0.10 kmole) and pH adjusted to 4-6 with aqueous sodium hydroxide. Sodium dithionite (10 kg, 0.06 kmole) is added, resulting in a decolourized process solution. Compound B is crystallized directly from this process solution by addition of seeds followed by cooling to 20-35°C, filtered, washed with water and dried. The azo dimer of Compound B is not detected in HPLC analysis of the product.

All patents, journal articles, publications and other documents discussed and/or cited above are hereby incorporated by reference.

## Claims

1. A process for purifying a solution of 5-amino-N,N'-bis(2,3-dihydroxypropyl)-2,4,6-triiodoisophthalamide (Compound B) in an industrial scale synthesis of iohexol, iodixanol or ioversol, comprising the step of adding 1 to 10 mole percent of sodium dithionite to the solution of Compound B at pH above 4 to convert azo dimer of Compound B to hydrazo dimer of Compound B, prior to crystallization of Compound B.
